# EUROPEAN PATENT APPLICATION

(11) **EP 1 939 301 A1**
(43) Date of publication of application: **02.07.2008**
(21) Application number: 06810702.8
(22) Date of filing: 28.09.2006
(51) Int. Cl.: C12P 1/04, C12N 15/09

(54) **PROCESS FOR PRODUCING USEFUL SUBSTANCE**

(30) Priority: 29.09.2005 JP 2005284540
(71) Applicant: KYOWA HAKKO KOGYO CO., LTD., Chiyoda-ku, Tokyo 100-8185 (JP)
(72) Inventor: HARA, Kiyotaka, Chiba 299-1162 (JP); KATO, Makiko, Tokyo 194-0022 (JP); MORI, Hideo, Tokyo 194-0021 (JP)
(74) Representative: Bohmann, Armin K.
(86) International application number: PCT/JP2006/319244
(87) International publication number: WO 2007/037301

(57) **Abstract**

The present invention provides processes of producing useful substances, which include the steps of culturing in a medium microorganisms that lack from their chromosomal DNA all or part of a gene encoding a protein having the amino acid sequence shown in SEQ ID NO: 1 or a gene encoding a protein that is 80% or more homologous to the amino acid sequence shown in SEQ ID NO: 1 so as to produce and accumulate the useful substances in the culture, and recovering the useful substances from the culture.

## Description

### Technical Field

The present invention relates to a process for producing a useful substance using a microorganism.

### Background Art

Many processes for producing useful substances by exploiting the ATP-supplying activity of microorganisms have been reported in the literature (Non-Patent Documents 1 to 4). However, while these reports examined in detail enzymes that produce useful substances using ATP, they fail to provide a detailed analysis of the complex enzymatic activity or bacterial activity involved in the supply of ATP. Rather, the above-mentioned reports merely mention that ATP is supplied through the energy generated from glucose catabolism; sufficient analysis of the complex enzymatic system involved in ATP supply has yet to be conducted.

The entire nucleotide sequences of the chromosomal DNA for many microorganisms have been determined (Non-Patent Document 5). Furthermore, methods for deleting a specific gene or a region from the chromosomal DNA of a microorganism, according to one's intentions, by using homologous recombination techniques are also known (Non-Patent Document 6). In addition, a library of one-gene-disruption mutants of almost all genes in a microorganism's chromosomal DNA, libraries of mutant strains in which deletable regions of about 20 kbp in the chromosomal DNA have been thoroughly and separately deleted, and the like have been prepared using the information of the entire nucleotide sequence of chromosomal DNA and homologous recombination techniques (Non-Patent Documents 7 and 8).

The *nlpD* gene product of *E. coli* has been shown to be a lipoprotein. The number of viable cells of *nlpD* gene-disrupted *E. coli* strain has been reported to decrease as compared to the wild-type strain when allowed to stand for a long period of time (Non-Patent Document 9). The *nlpD* gene-disrupted *E. coli* mutant strain is available from the Nara Institute of Science and Technology (Non-Patent Document 10).

However, it is not presently known that microorganisms lacking the *nlpD* gene are useful in producing useful substances.
Non-Patent Document 1: Appl. Microbiol. Biotechnol. (Applied Microbiology and Biotechnology), 48, 693 (1997)
Non-Patent Document 2: Biosci. Biotechnol. Biochem. (Bioscience, Biotechnology and Biochemistry), 61, 960 (1997)
Non-Patent Document 3: Nature Biotechnol. (Nature Biotechnology), 16, 847 (1998)
Non-Patent Document 4: J. Appl. Biochem. (Journal of Applied Biochemistry), 5, 43 (1983)
Non-Patent Document 5: http://www.tigr.org/tdb/mdb/mdbcomplete.html
Non-Patent Document 6: J. Bacteriol. (Journal of Bacteriology), 180, 2063 (1998)
Non-Patent Document 7: J. Biochem. Mol. Biol. (Journal of Biochemistry and Molecular Biology), 37, 83 (2004)
Non-Patent Document 8: Nature Biotechnol. (Nature Biotechnology), 20, 1018 (2002)
Non-Patent Document 9: J. Bacteriol. (Journal of Bacteriology), 176, 1630 (1994)
Non-Patent Document 10: http://ecoli.aist-nara.ac.jp/GB5/search.jsp

### Disclosure of the Invention

### [Problems to be Solved by the Invention]

An objective of the present invention is to provide a process for efficiently producing a useful substance using ATP as an energy source.

### [Means for Solving the Problems]

The present invention relates to following (1) to (7):
(1) A process for producing a useful substance, which comprises culturing a microorganism that lacks from its chromosomal DNA all or part of a gene encoding a protein comprising the amino acid sequence of SEQ ID NO: 1 or a gene encoding a protein that has 80% or more homology to the amino acid sequence of SEQ ID NO: 1 in a medium so as to produce and accumulate the useful substance in the culture, and recovering the useful substance from the culture;
(2) A process for producing a useful substance, which comprises allowing a culture or a treated the culture of a microorganism that lacks from its chromosomal DNA all or part of a gene encoding a protein comprising the amino acid sequence of SEQ ID NO: 1 or a gene encoding a protein that has 80% or more homology to the amino acid sequence of SEQ ID NO: 1, an enzyme having an activity to produce a useful substance using ATP as an energy source or a culture or a treated culture of a cell having the activity, a carbon source, and a precursor substance of the useful substance to coexist in a medium so as to produce and accumulate the useful substance in the medium, and recovering the useful substance from the medium;
(3) The process according to above-described (1) or (2), wherein the gene encoding a protein comprising the amino acid sequence of SEQ ID NO: 1 has the nucleotide sequence of SEQ ID NO: 2;
(4) The process according to any one of above-described (1) to (3), wherein the ability of the microorganism or cell to produce the useful substance has been improved by a method selected from the following [1] to [5]:
   [1] a method of partially or completely releasing at least one of the mechanisms that regulate the biosynthesis of the useful substance;
   [2] a method of enhancing expression of at least one of the enzymes involved in the biosynthesis of the useful substance;
   [3] a method of increasing the copy number of at least one of the enzyme genes involved in the biosynthesis of the useful substance;
   [4] a method of partially or completely blocking at least one of the branched metabolic pathways of the biosynthetic pathway of the useful substance to metabolites other than the useful substance; and
   [5] a method of selecting a cell strain that is more resistant to an analog of the useful substance as compared with a wild type strain;
(5) The process according to any one of above-described (1) to (4), wherein the microorganism belongs to the genus *Azotobacter, Erwinia, Escherichia, Klebsiella, Methylobacillus, Pseudomonas,* or *Salmonella;*
(6) The process according to any one of above-described (1) to (5), wherein the microorganism is a microorganism of the species *Azotobacter vinelandii, Erwinia carotovora, Escherichia coli, Klebsiella pneumoniae, Methylobacillus flagellatus, Pseudomonas fluorescens, Pseudomonas putida,* and *Salmonella thypimurium;* and
(7) The process according to any one of above-described (1) to (6), wherein the useful substance is selected from the group consisting of a protein, peptide, amino acid, nucleic acid, vitamin, sugar, sugar alcohol, alcohol, organic acid, biologically active low-molecular-weight compound, and lipid.

### [Effects of the Invention]

Useful substances that are produced using ATP as an energy source can be efficiently produced according to the present invention.

### Best Mode for Carrying out the Invention

### 1. Microorganisms used in the production process of the present invention:

Microorganisms used in the production process of the present invention include microorganisms that lack from their chromosomal DNA all or part of a gene encoding a protein comprising the amino acid sequence shown in SEQ ID NO: 1 (hereinafter also referred to as the NlpD protein) or a gene encoding a protein having 80% or higher, preferably 90% or higher, more preferably 95% or higher, even more preferably 97% or higher, still more preferably 98% or higher, and yet more preferably 99% or higher homology to the amino acid sequence shown in SEQ ID NO: 1 (hereinafter also referred to as an NlpD homologue protein). A "gene" described herein includes a structural gene, and regions having particular regulatory function, such as promoter and operator.

Homology of amino acid and nucleotide sequences can be determined using the algorithm BLAST by Karlin and Altschul [Pro. Natl. Acad. Sci. USA, 90, 5873 (1993)] and the algorithm FASTA [Methods Enzymol., 183, 63 (1990)]. Programs called BLASTN and BLASTX have been developed based on this algorithm, BLAST [J. Mol. Biol., 215, 403 (1990)]. When nucleotide sequences are analyzed by BLASTN based on BLAST, the parameters may be set, for example, as score = 100 and wordlength =12. When amino acid sequences are analyzed by BLASTX based on BLAST, the parameters may be set, for example, as score = 50 and wordlength = 3. When BLAST and Gapped BLAST programs are used, default parameters are used for each program. Specific procedures for these analytical methods are well known (http://www.ncbi.nlm.nih.gov.).

Microorganisms that lack all or part of a gene encoding an NlpD protein or a gene encoding an NlpD homologue protein (hereinafter also referred to as an *nlpD* gene or a homologue gene thereof) include:
(1) microorganisms that do not express the protein encoded by the *nlpD* gene or a homologue gene thereof as a result of failure of transcriptional regulation, for example by a promoter or an operator of an *nlpD* gene or a homologue gene thereof, due to the deletion of nucleotide(s) from the nucleotide sequence of the gene in the microorganism's chromosomal DNA;
(2) microorganisms that do not express the NlpD protein or a homologue protein thereof as an active protein as a result of a frameshift occurring within the structural gene of the *nlpD* gene or a homologue gene thereof due to the deletion of nucleotide(s) from the nucleotide sequence of the gene in the microorganism's chromosomal DNA; and
(3) microorganisms that do not express an active protein due to the fact that they lack all or part of a structural gene from the *nlpD* gene or a homologue gene thereof as a result of the deletion of nucleotide(s) from the nucleotide sequence of the gene in the microorganism's chromosomal DNA. Preferred microorganisms are those of (3).

More specifically, the microorganisms described above in (3) include microorganisms that do not express an active protein because all or part of a structural gene in the nucleotide sequence of SEQ ID NO: 2 is deleted. A partial deletion includes a single nucleotide deletion within the nucleotide sequence of SEQ ID NO: 2, so long as the deletion results in the loss of an activity of the protein comprising the amino acid sequence of SEQ ID NO: 1. Preferred deletions are those of 5 to 100 nucleotides within the structural gene, more preferably those of 10 to 100 nucleotides within the gene, and even more preferably those of 50 to 100 nucleotides within the gene.

Microorganisms to be used in the production process of the present invention may belong to any genus, so long as they lack all or part of an *nlpD* gene or a homologue gene thereof in their chromosomal DNA as described above. Preferred microorganisms are prokaryotes. More preferred microorganisms are bacteria.

Examples of such bacteria include microorganisms belonging to the *Azotobacter* genus, *Erwinia* genus, *Escherichia* genus, *Klebsiella* genus, *Methylobacillus* genus, *Pseudomonas* genus, or *Salmonella* genus; and preferably are *Azotobacter vinelandii, Erwinia carotovora, Escherichia coli, Klebsiella pneumoniae, Methylobacillus flagellatus, Pseudomonas fluorescens, Pseudomonas putida, Salmonella thypimurium,* and the like; and more preferably are *E*. *coli.*

### 2. Preparation of microorganisms useful in the production process of the present invention:

Microorganisms useful in the production process of the present invention can be prepared by:
(1) deleting all or part of an *nlpD* gene or a homologue gene thereof from the chromosomal DNA of a microorganism having the ability to produce a useful substance;
   or
(2) conferring the ability to produce useful substances to a microorganism that lacks all or part of an *nlpD* gene or a homologue gene thereof in the chromosomal DNA.

Microorganisms having the ability to produce useful substances include any microorganism, so long as they have the ability to produce one or more types of useful substances. Examples of such microorganisms include strains isolated from nature that themselves have the desired ability as well as microorganisms in which the ability to produce desired useful substances is artificially conferred by known methods. Such methods include:
(a) methods of partially or completely releasing at least one of the mechanisms that regulate the biosynthesis of useful substances;
(b) methods of potentiating the expression of at least one of the enzymes involved in the biosynthesis of useful substances;
(c) methods of increasing the copy number of at least one of the enzyme genes involved in the biosynthesis of useful substances;
(d) methods of partially or completely blocking at least one of the branched metabolic pathways of the biosynthetic pathway of a useful substance to metabolites other than the useful substance; and
(e) methods of selecting a cell strain that is more resistant to an analog of a useful substance as compared with a wild-type strain.

The known methods described above can be used alone or in combination.

Specifically, when the useful substance is an amino acid, for example, the methods listed above in (a) to (e) include: the example of method (a) described in Agric. Biol. Chem., 43, 105-111 (1979); J. Bacteriol., 110, 761-763 (1972); Appl. Microbiol. Biotechnol., 39, 318-323(1993); and others; the example of method (b) described in Agric. Biol. Chem., 43, 105-111 (1979); J. Bacteriol., 110, 761-763 (1972); and others; the example of method (c) described in Appl. Microbiol. Biotechnol., 39, 318-323 (1993); Agric. Biol. Chem., 39, 371-377 (1987); and others; the example of method (d) described in Appl. Environ. Microbiol., 38, 181-190 (1979); Agric. Biol. Chem., 42, 1773-1778 (1978); and others; the example of method of (e) described in Agric. Biol. Chem., 36, 1675-1684 (1972); Agric. Biol. Chem., 41, 109-116 (1977); Agric. Biol. Chem., 37,2013-2023 (1973); Agric. Biol. Chem., 51, 2089-2094 (1987); and others. Microorganisms having the ability to produce and accumulate various amino acids can be prepared according to the protocols described in the documents listed above and others.

Many illustrative examples of methods for preparing microorganisms having the ability to produce and accumulate amino acids, according to an above method of (a) to (e) or a combination thereof, are described in Biotechnology 2nd Ed., Vol. 6, Products of Primary Metabolism (VCH Verlagsgesellschaft mbH, Weinheim, 1996) section 14a and 14b; Advances in Biochemical Engineering/Biotechnology 79, 1-35 (2003); and Aminosan Hakkou (Amino acid fermentation), Gakkai Shuppan Center (Japan Scientific Societies Press), Hiroshi Aida et al., (1986). In addition to the above, there are many reports of methods for preparing microorganisms having the ability to produce and accumulate specific amino acids: JP-A (Kokai) 2003-164297; Agric. Biol. Chem., 39, 153-160 (1975); Agric. Biol. Chem., 39, 1149-1153 (1975); JP-A (Kokai) S58-13599; J. Gen. Appl. Microbiol., 4, 272-283 (1958); JP-A (Kokai) S63-94985; Agric. Biol. Chem., 37, 2013-2023 (1973); WO 97/15673; JP-A (Kokai) S56-18596; JP-A (Kokai) S56-144092; JP-A (Kohyo) 2003-511086; and others. Microorganisms having the ability to produce one or more types of amino acids can be prepared according to the protocols described in the documents listed above and others.

There are also many reports of methods for conferring the ability to produce useful substances other than amino acids to microorganisms. Any known method can be used to prepare microorganisms to be used in the production process of the present invention.

Microorganisms that lack all or part of an *nlpD* gene or a homologue gene thereof in their chromosomal DNA can be obtained, for example, by introducing nucleotide deletions into the *nlpD* gene or a homologue gene thereof in the chromosomal DNA of the microorganism, using the nucleotide sequence information for a gene that encodes a protein comprising the amino acid sequence shown in SEQ ID NO: 1 and that is contained in the chromosomal DNA of the microorganism listed below or the nucleotide sequence information for a homologue gene of the *nlpD* gene; however, there is no limitation on the methods, so long as the microorganisms can be obtained by such a method.

The nucleotide sequence of a homologue gene of an *nlpD* gene can be determined by identifying and obtaining a homologue gene of the *nlpD* gene via Southern hybridization with the chromosomal DNA of various microorganisms, using as a probe all or part of the structural gene in the nucleotide sequence as set forth in SEQ ID NO: 2, or via PCR, using as primer DNAs designed based on the nucleotide sequence of SEQ ID NO: 2 and using as templates chromosomal DNAs of various microorganisms; and then analyzing the nucleotide sequence of the gene according to a conventional method.

Chromosomal DNAs that are subjected to Southern hybridization or PCR may be derived from any microorganisms. Such chromosomal DNAs preferably include those of microorganisms belonging to the genus *Azotobacter, Erwinia, Escherichia, Klebsiella, Methylobacillus, Pseudomonas,* or *Salmonella,* and more preferably those of *Azotobacter vinelandii, Erwinia carotovora, Escherichia coli, Klebsiella pneumoniae, Methylobacillus flagellatus, Pseudomonas fluorescens, Pseudomonas putida,* or *Salmonella thypimurium.*

The "hybridization" described above means that a DNA hybridizes to another DNA having a specific nucleotide sequence, or a portion thereof. Thus, a DNA having a specific nucleotide sequence or a portion thereof can be used as a probe in northern or Southern blot analysis, or as an oligonucleotide primer in PCR analysis. DNAs used as probes include DNAs of at least 100 nucleotides or more, preferably 200 nucleotides or more, and more preferably 500 nucleotides or more. DNAs used as primers include DNAs of at least 10 nucleotides or more, preferably 15 nucleotides or more.

Methods of DNA hybridization experiments are well known. For example, those skilled in the art can determine hybridization conditions according to the present specification. Examples of such hybridization conditions are described in Molecular Cloning, 2nd Ed., or 3rd Ed., (2001); Methods for General and Molecular Bacteriology, ASM Press (1994); Immunology methods manual, Academic press (Molecular); and others. Such methods can be conducted according to protocols described in many other standard textbooks as well.

Hybridization is preferably carried out under stringent conditions. A preferred stringent condition is: overnight incubation at 42°C of probe DNA and filter with immobilized DNA in a solution containing 50% formamide, 5x SSC (750 mM sodium chloride and 75 mM sodium citrate), 50 mM sodium phosphate (pH 7.6), 5x Denhardt's solution, 10% dextran sulfate, and 20 µg/l denatured salmon sperm DNA, followed by, for example, washing of the filter in a solution of 0.2x SSC at about 65°C. Lower stringency conditions may also be used. Stringent conditions can be altered by adjusting the formamide concentration (the stringency becomes lower as the formamide concentration is lowered), or by altering salt concentrations or temperature conditions. An example of a low stringency condition includes overnight incubation at 37°C in a solution containing 6x SSCE (20x SSCE contains 3 mol/l sodium chloride, 0.2 mol/l sodium dihydrogenphosphate, and 0.02 mol/l EDTA (pH 7.4)), 0.5% SDS, 30% formamide, and 100 µg/l denatured salmon sperm DNA, followed by washing with 1x SSC, 0.1% SDS solution at 50°C. Even lower stringency conditions include hybridization under the above low stringency condition, with the exception that a solution with high salt concentration (for example, 5x SSC) is used, followed by washing.

The various conditions described above can be also set by adding or changing a blocking reagent that suppresses the background in hybridization experiments. The addition of a blocking reagent as described above may be accompanied by the alteration of a hybridization condition for conditional compatibility.

DNAs that can hybridize under the stringent conditions described above include DNAs having at least 90% or higher, preferably 95% or higher, more preferably 97% or higher, even more preferably 98% or higher, still more preferably 99% or higher homology to the nucleotide sequence of SEQ ID NO: 2, such homology calculated, for example, based on the above-described parameters using the above-described BLAST, FASTA, or the like.

Specifically, the nucleotide sequences of homolog genes of the *nlpD* gene include the nucleotide sequences of the *nlpD* genes derived from *Azotobacter vinelandii* (Genbank accession no. AF421351), *Erwinia carotovora* (Genbank accession no. BX950851), *Pseudomonas jluorescens* (Genbank accession no. AF245440), *Pseudomonas putida* (Genbank accession no. AF260132), and *Salmonella thypimurium* (Genbank accession no. AE008833).

Methods of introducing nucleotide deletions into the genes of the chromosomal DNA of a microorganism include methods that utilize homologous recombination. Conventional methods using homologous recombination include methods that use plasmids for homologous recombination, such plasmids produced, for example, by ligating a mutant gene introduced with a nucleotide deletion to a plasmid DNA carrying a drug resistance gene which cannot replicate by itself in the host cells to which the deletion is to be introduced.

After the plasmid for homologous recombination is introduced into host cells by conventional methods, transformed strains in which the plasmid for homologous recombination has been integrated into the chromosomal DNA through homologous recombination may be selected using drug resistance as an indicator. The obtained transformed strains can be cultured in a medium without the drug for several hours to one day, and then applied onto agar medium containing the drug and agar medium without the drug. Strains that do not grow in the former medium but do grow in the latter medium can then be selected to obtain strains whose chromosomal DNAs have undergone a second homologous recombination. Whether a nucleotide deletion has been introduced into a gene of interest in the chromosomal DNA can be confirmed by determining the nucleotide sequence of the region in the chromosomal DNA in which the gene introduced with the deletion and the like is present.

Microorganisms to which a nucleotide deletion can be introduced into a gene of interest within their chromosomal DNAs by the methods described above include, for example, microorganisms belonging to the genus *Escherichia.*

Furthermore, methods for efficiently introducing nucleotide deletions into multiple genes, via homologous recombination, include methods that use linear DNAs.

Specifically, in such methods, cells are allowed to incorporate a linear DNA that contains regions present at both outer sides of the target region of the chromosomal DNA into which a nucleotide deletion is to be introduced, and allowed to undergo homologous recombination between the chromosomal DNA and the introduced linear DNA. Such methods are applicable to any microorganism, so long as they efficiently incorporate linear DNAs. Such microorganisms preferably include those belonging to the genus *Escherichia,* more preferably *Escherichia coli,* even more preferably *Escherichia coli* expressing a group of λ phage-derived recombination proteins (the λRed recombination system).

Examples of *Escherichia coli* that express the λRed recombination system include the *Escherichia coli* JM101 strain having pKD46 [available from the *Escherichia coli* Genetic Stock Center (Yale University, U.S.A)], which is a plasmid DNA carrying the λRed recombination system gene.

DNAs used in homologous recombination may include:
(a) linear DNA comprising DNAs present at both outer sides of the target region of the chromosomal DNA into which a nucleotide deletion is to be introduced or DNAs having homology to the DNAs arranged at both ends of a drug resistance gene;
(b) linear DNA in which DNAs present at both outer sides of the target region of the chromosomal DNA into which a nucleotide deletion is to be introduced or DNAs having homology to the DNAs are directly linked together;
(c) linear DNA comprising DNAs present at both outer sides of the target region of the chromosomal DNA into which a nucleotide deletion is to be introduced or DNAs having homology to the DNAs arranged at both ends of a DNA comprising a drug resistance gene and a gene that can be used for negative selection; and
(d) the linear DNA described above in (a), which further comprises a DNA comprising a nucleotide sequence recognized by yeast-derived Flp recombinase [Proc. Natl. Acad. Sci. USA., 82, 5875 (1985)] between the drug resistance gene and the DNAs present at both outer sides of the target region of the chromosomal DNA into which a nucleotide deletion is to be introduced or DNAs having homology to the DNAs.

Any drug resistance gene can be used, so long as it can confer resistance to a drug to which the host microorganism is susceptible. When *Escherichia coli* is used as a host microorganism, examples of drug resistance genes include the kanamycin resistance gene, chloramphenicol resistance gene, gentamycin resistance gene, spectinomycin resistance gene, tetracycline resistance gene, and ampicillin resistance gene.

Genes that can be used for negative selection include genes that are lethal to a host microorganism under specific culture conditions when the genes are expressed in the microorganism. Examples of such genes include the *sacB* gene [Appl. Environ. Microbiol., 59, 1361-1366 (1993)] derived from microorganisms belonging to the genus *Bacillus* and the *rpsL* gene [Genomics, 72, 99-104 (2001)] derived from microorganisms belonging to the genus *Escherichia.*

The above-mentioned DNAs present at both ends of the linear DNA, which are DNAs present outside of both ends of a target region of a chromosomal DNA into which a nucleotide deletion is to be introduced, or DNAs having homology and homology to such DNAs, may be arranged in the linear DNA in the same direction as that of the chromosomal DNA. Their length is preferably about 10 bp to 100 bp, more preferably about 20 bp to 50 bp, and even more preferably about 30 bp to 40 bp.

The nucleotide sequence recognized by yeast-derived Flp recombinase is not particularly limited, so long as the protein recognizes the nucleotide sequence and catalyzes homologous recombination. Such a nucleotide sequence preferably includes a DNA comprising the nucleotide sequence shown in SEQ ID NO: 3 as well as DNAs comprising the nucleotide sequence in which one to several nucleotides in the nucleotide sequence of SEQ ID NO: 3 are deleted, substituted, or added, and the resulting DNA is recognized by yeast-derived Flp recombinase and whose homologous recombination is catalyzed by the recombinase.

The phrase "having homology" means that the above-described linear DNA has homology that allows homologous recombination in a region of interest in the chromosomal DNA. Specifically, such homology include 80% or higher, preferably 90% or higher, more preferably 95% or higher, and even more preferably 100% homology.

The above-described nucleotide sequence homology can be determined using programs such as BLAST and FASTA as described above.

The above-described linear DNA can be produced by PCR. Alternatively, linear DNAs of interest can also be obtained by constructing a DNA including the above-described linear DNA with a plasmid and treating it with restriction enzymes.

Methods for introducing a nucleotide deletion, substitution, or addition into the chromosomal DNA of a microorganism include the following methods 1 to 4.

### Method 1:

A method in which the linear DNA described above in (a) or (d) is introduced into host microorganisms to yield transformed strains in which the linear DNA has been inserted into the chromosomal DNA through homologous recombination that may be selected using drug resistance as an indicator.

### Method 2:

A method in which the linear DNA described above in (b) is introduced into transformed strains obtained by the method 1 described above and a region in the chromosomal DNA of the microorganism is substituted or deleted by removing the drug gene inserted into the chromosomal DNA by the method.

### Method 3:

A method in which:
[1] the linear DNA described above in (c) is introduced into host microorganisms and transformed strains in which the linear DNA has been inserted into the chromosomal DNA through homologous recombination are selected using drug resistance as an indicator;
[2] DNAs in which DNAs having homology to DNAs positioned outside of both ends of a region targeted for a nucleotide substitution or deletion in the chromosomal DNA are arranged in the same order as in the chromosomal DNA are synthesized, and introduced into the transformed strains obtained above in [1]; and
[3] the transformed strains treated as described above in [2] are cultured under a condition in which a gene applicable to negative selection is expressed, and strains that can grow in the culture are selected as strains in which the drug resistance gene and the gene applicable to negative selection have been removed from the chromosomal DNA.

### Method 4:

A method in which:
[1] the linear DNA described above in (d) is introduced into host microorganisms and transformed strains in which the linear DNA has been inserted into the chromosomal DNA through homologous recombination are selected using drug resistance as an indicator; and
[2] an expression plasmid for Flp recombinase gene is introduced into transformed strains obtained above in [1] to express the gene, and then strains sensitive to the drug used in [1] above are obtained.

Any method of introducing linear DNAs into host microorganisms may be used in the methods described above, so long as the method allows for the introduction of DNA into microorganisms. Such methods include, for example, methods using calcium ion [Proc. Natl. Acad. Sci., USA, 69, 2110 (1972)], protoplast method (JP-A (Kokai) S63-2483942), and electroporation method [Nucleic Acids Res., 16, 6127 (1988)].

An arbitrary gene can be inserted into the chromosomal DNA simultaneously upon removal of the drug resistance gene and the like using the linear DNA of Method 2 or Method 3 [2] in which an arbitrary gene to be inserted into the chromosomal DNA is integrated near the center of the linear DNA.

In Methods 2 to 4 described above, foreign genes, such as the drug resistance gene and a gene applicable to negative selection, will not remain in the chromosomal DNAs of the finally obtained transformed strains. Therefore, by using the methods, microorganisms having nucleotide deletions, substitutions, or additions in two or more regions at different positions in the chromosomal DNA can be readily produced by repeating the procedure of the methods using the same drug resistance gene and gene applicable to negative selection.

### 3. Process for producing useful substances of the present invention:

In the context of the present invention, a useful substance produced by the production process herein may be any substance of interest, so long as it is an industrially useful substance arid it directly or indirectly requires ATP for its biosynthesis when produced by utilizing ATP and metabolic ability of microorganisms or cells. Such substances preferably include proteins, peptides, amino acids, nucleic acids, vitamins, sugars, sugar alcohols, alcohols, organic acids, biologically active low-molecular-weight compounds, and lipids. More preferably, such proteins include inosine kinase, glutamate 5-kinase (EC 2.7.2.11), glutamate-5-semialdehyde dehydrogenase (EC 1.2.1.41), pyrroline-5-carboxylate reductase (EC 1.5.1.2), γ-glutamylcysteine synthetase (EC 6.3.2.2), glutathione synthetase (EC 6.3.2.3), human granulocyte colony-stimulating factor, xylose reductase, and P450; peptides include glutathione; amino acids include L-alanine, glycine, L-glutamine, L-glutamic acid, L-asparagine, L-aspartic acid, L-lysine, L-methionine, L-threonine, L-leucine, L-valine, L-isoleucine, L-proline, L-histidine, L-arginine, L-tyrosine, L-tryptophan, L-phenylalanine, L-serine, L-cysteine, L-3-hydroxyproline, and L-4-hydroxyproline; nucleic acids include inosine, guanosine, inosinic acid, and guanylic acid; vitamins include riboflavin, thiamine, and ascorbic acid; sugars include xylose; sugar alcohols include xylitol; alcohols include ethanol; organic acids include lactic acid and succinic acid; biologically active low-molecular-weight compounds include glutathione; and lipids include eicosapentaenoic acid (EPA) and docosahexaenoic acid (DHA).

Examples of processes of the present invention for producing useful substances include: <1> processes that comprise the steps of culturing a microorganism described in section 2 above in a culture medium so as to produce and accumulate the useful substances in the culture, and recovering the useful substances from the culture and <2> processes that comprise the steps of allowing a culture or a treated culture of a microorganism described in section 2 above, an enzyme having the activity of producing useful substances using ATP as an energy source or a culture or a processed product of a culture of cells having such an activity, carbon source, and precursor substances of the useful substances to coexist in a medium so as to produce and accumulate the useful substances in the medium, and recovering the useful substances from the medium.

### (1) Production of useful substances by fermentation processes

In the processes described above in <1>, the microorganism can be cultured in a culture medium according to conventional methods known for culturing microorganisms.

Specifically, both natural media and synthetic media may be used, so long as they contain a carbon source that can be assimilated by the microorganisms, a nitrogen source, inorganic salts, and the like and allow for the efficient culture of the microorganisms.

Any carbon sources may be used, so long as they can be assimilated by the microorganisms; examples include glucose, fructose, sucrose, molasses containing them, carbohydrates such as starch and starch hydrolysates, organic acids such as acetic acid and propionic acid, and alcohols such as ethanol and propanol.

As nitrogen sources, ammonia; ammonium chloride; ammonium salts of inorganic and organic acids such as ammonium sulfate, ammonium acetate, and ammonium phosphate; other nitrogen-containing compounds; peptone, meat extract, yeast extract, corn steep liquor, casein hydrolysate, soybean cake, soybean cake hydrolysate, and various bacterial cells from fermentation broth; digests thereof; and the like can be used.

As inorganic salts, monopotassium phosphate, dipotassium phosphate, magnesium phosphate, magnesium sulfate, sodium chloride, ferrous sulfate, manganese sulfate, copper sulfate, calcium carbonate, and the like can be used.

Generally, culture is achieved under aerobic conditions, including the steps of shaking culture or stirring culture with aeration. Preferred culture temperatures range from 15 to 40°C. In general, the culture period is five hours to seven days. The pH is kept at 3.0 to 9.0 during culture. The pH may be adjusted using an inorganic or organic acid, alkaline solution, urea, calcium carbonate, ammonia, and the like.

If necessary, antibiotics, such as ampicillin and tetracycline, may be added to the medium during culture.

When microorganisms transformed with an expression vector having an inducible promoter are cultured, an inducer may be added to the medium if needed. For example, when microorganisms transformed with an expression vector having the *lac* promoter are cultured, isopropyl-β-D-thiogalactopyranoside or the like may be added to the medium; and when microorganisms transformed with an expression vector having the *trp* promoter are cultured, indoleacrylic acid or the like may be added to the medium.

Useful substances produced and accumulated in a culture can be collected according to conventional methods, using for example activated carbon or ion exchange resins, or by extraction using organic solvents, recrystallization, thin layer chromatography, high performance liquid chromatography, or the like.

### (2) Process using as an ATP source a microorganism described above in section 2:

The culture of a microorganism described above in section 2, which is to be used in the process described above in <2>, can be obtained by culturing the microorganism described above in section 2 by the culture method described above in (1).

The treated culture of a microorganism is not particularly limited, so long as it has the activity of producing ATP. Such treated culture includes, for example, a concentrated culture, a dried culture, a freeze-dried culture, products obtained by reducing the osmotic pressure of the culture, cells of the microorganism obtained by centrifuging the culture; products of the cells, which retain the morphology of the cells and have substantially the same activity as that of the culture, such as dried products of the cells, freeze-dried products of the cells, products of the cells resulting from detergent treatment, products of the cells resulting from enzyme treatment, products of the cells resulting from solvent treatment, and immobilized products of the cells; and crude enzyme extracts, such as sonication products of the cells and mechanically-disintegrated products of the cells.

Suitable media include water, aqueous media, organic solvents, and mixture of water or an aqueous medium and an organic solvent. Examples of aqueous media include buffers such as phosphate buffer, HEPES (N-2-hydroxyethyl piperazine-N-ethanesulfonic acid) buffer, and Tris [Tris(hydroxymethyl)aminomethane] hydrochloride buffer. Any organic solvents can be used, as long as they do not inhibit the reaction. Such organic solvents include, for example, acetone, ethyl acetate, dimethylsulfoxide, xylene, methyl alcohol, ethyl alcohol, and butanol. Furthermore, the aqueous media also include culture media used to culture the microorganisms described above in (1) and supernatants of cultures obtained by culturing the microorganisms described above in (1).

Enzymes having the activity of producing useful substances using ATP as an energy source are not particularly limited, so long as ATP is essential for the reaction catalyzed by them. Examples of such enzymes include γ-glutamylcysteine synthetase and glutathione synthetase. Furthermore, cells that have the activity of producing useful substances using ATP as an energy source are not particularly limited, so long as they have a metabolic system in which ATP is directly or indirectly involved in the process of producing the useful substances through metabolizing precursors of the useful substances. Examples of such cells include microorganisms, animal cells, plant cells, and insect cells, and preferably are microorganisms. Such microorganisms are preferably those to which the ability to produce desired useful substances has been artificially conferred by the method described above in section 2.

The treated culture of the cells is not particularly limited, so long as it can produce useful substances using ATP. Examples of such treated culture include a concentrated culture, a dried culture, a freeze-dried culture, products obtained by reducing the osmotic pressure of the culture, cells of the microorganism obtained by centrifuging the culture; products of the cells, which retains the morphology of the cells and has substantially the same activity as that of the culture, such as dried products of the cells, freeze-dried products of the cells, products of the cells resulting from detergent treatment, products of the cells resulting from enzyme treatment, products of the cells resulting from solvent treatment, and immobilized products of the cells; and crude enzyme extracts such as sonication products of the cells, mechanically-disintegrated products of the cells, protein fractions extracted from the cells, and immobilized enzymes.

Carbon sources are not particularly limited, so long as microorganisms to be used in the production process of the present invention can metabolize and use them to generate ATP. Examples of such carbon sources include glucose, fructose, sucrose, molasses containing them, carbohydrates such as starch and starch hydrolysates, organic acids such as acetic acid and propionic acid, and alcohols such as ethanol and propanol.

Precursors of useful substances are not particularly limited, so long as they are metabolized within cells and converted into useful substances, and can be appropriately selected from substances upstream of a known pathway of biosynthesis of a desired useful substance.

In the production process described above in <2>, phosphoric acid may be added to a medium, if needed.

Furthermore, a surfactant such as Triton X-100, benzalkonium chloride, and sodium dodecyl sulfate may be added to the medium, if necessary. Concentration of a surfactant may be any concentration, so long as the production of useful substances is not inhibited. The concentration is 0.05 to 5%, preferably 0.1 to 2%, and more preferably 0.2 to 1%.

The amount of microorganisms or cells having the activity of producing useful substances using ATP as an energy source, which are to be used in the production methods described above in <2>, varies depending on their specific activity and the like. However, 5 to 1000 mg, preferably 10 to 400 mg may be added in wet weight per 1 mg of precursor of a useful substance, for example. An enzyme having the activity of producing useful substances using ATP as an energy source may be added in an amount of 0.01 to 100 mg, preferably 0.1 to 10 mg per 1 mg of precursor of a useful substance. Reactions are preferably conducted at 20 to 50°C, more preferably at 25 to 37°C. Reaction times vary depending on the amount, specific activity, and the like of an enzyme source to be used; however, the time is generally 2 to 150 hours, and preferably 6 to 120 hours.

Useful substances can be recovered from media by the methods described above in (1).

Examples are described below; however, the present invention should not be construed as being limited thereto. Methods described in "Molecular Cloning, A Laboratory Manual, Third Edition, Cold Spring Harbor Laboratory Press (2001)" can be appropriately used for DNA manipulation or the like as described in the Examples below.

### [Example 1] Preparation of strains lacking the nlpD gene

*E. coli* BW25113 *ΔnlpD*::*km* (hereinafter referred to as "*E*. *coli* ΔnlpD-K strain") in which the *nlpD* gene is disrupted with a kanamycin resistance marker was obtained from the Nara Institute of Science and Technology.

PCR was carried out using a primer set of DNAs each consisting of the nucleotide sequence of SEQ ID NO: 4 or 5 and as a template the chromosomal DNA of *E. coli* CGSC7465 strain (available from the Coli Genetic Stock Center managed by Yale University, U.S.A.) having transposon Tn10 in the chromosomal DNA. 100 µl of a reaction solution containing 10 ng of the chromosomal DNA and 50 pmol each of primer DNAs was prepared according to the instructions appended to LA-Taq (TaKaRa Bio Inc.), and PCR was carried out using LA-Taq under the following conditions: keeping the temperature at 94°C for two minutes, followed by 30 cycles of 94°C for 15 seconds, 55°C for 20 seconds, and 68°C for 90 seconds, and subsequent incubation at 72°C for ten minutes.

After confirming the amplification of a DNA fragment of interest using agarose gel electrophoresis, the DNA fragment was excised from gel and purified. A DNA fragment having the tetracycline resistance gene in the middle and sequences homologous to the 5' and 3' end of the *nlpD* gene at the two ends was amplified by PCR using 1 µl of solution of the purified DNA as a template and a primer set of DNAs each consisting of the nucleotide sequence of SEQ ID NO: 6 or 7. 50 µl of a reaction solution containing 1 ng of template and 40 pmol each of primer DNAs was prepared according to the instructions appended to LA-Taq, and PCR was carried out using LA-Taq under the following conditions: keeping the temperature at 94°C for two minutes, followed by 30 cycles of 94°C for 15 seconds, 55°C for 20 seconds, and 68°C for two minutes, and subsequent incubation at 72°C for ten minutes. The amplified DNA fragment was purified using QIA quick PCR Purification Kit (QIAGEN) to give 30 µl of a DNA solution.

Competent cells of *E. coli* BW25113/pKD46 strain (available from the Coli Genetic Stock Center managed by Yale University, U.S.A.) were prepared by the method described in a document [Gene, 246, 321-330 (2000)]. Transformation was performed using 0.5 µl of the DNA solution prepared in the above. Transformation was carried out by electroporation using 0.1-cm cuvettes (BioRad) under the conditions of 1.8 KV and 25 µF.

Transformed cells were cultured using 1 ml of liquid LB medium [which contains 10 g/l Bacto tryptone (Difco), 5 g/l Bacto yeast extract (Difco), and 5 g/l sodium chloride, and to which 1 mol/l sodium hydroxide was added at the proportion of 1 ml/l] at 30°C for two hours. Then, the cells were applied onto LB agar plates (liquid LB medium containing 1.5% agar) containing 15 µg/ml tetracycline, 100 µg/ml ampicillin, and 1% glucose and incubated at 30°C overnight. The strain that included the tetracycline resistance gene in place of the *nlpD* gene in the chromosomal DNA of *E. coli* BW25113/pKD46 strain [*E. coli* BW25113/pKD46 *ΔnlpD*::*tet*] was then grown. This strain was named *E*. *coli* ΔnlpD-T.

Next, a P1 phage stock was prepared from *E*. *coli* ΔnlpD-T strain by the method described below. Cells of *E*. *coli* ΔnlpD-T strain were cultured at 30°C overnight using liquid LB medium [which contains 10 g/l Bacto tryptone (Difco), 5 g/l Bacto yeast extract (Difco), and 5 g/l sodium chloride, and to which 1 mol/l sodium hydroxide was added at the proportion of 1 ml/l] containing 15 µg/ml tetracycline and 100 µg/ml ampicillin. Then, a 0.5-ml aliquot of the culture was taken out; 1.4 ml of fresh liquid LB medium and 100 µl of 0.1 mol/l calcium chloride were added to it; and they were mixed.

After the mixture was cultured with shaking at 30°C for five to six hours, 400 µl of the mixture was transferred into a sterile tube and 2 µl of the P1 phage stock solution was added thereto. After keeping the mixture at 37°C for ten minutes, 3 ml of a soft agar solution (which contains 10 g/l Bacto tryptone, 5 g/l Bacto yeast extract, 5 mmol/l calcium chloride, and 15 g/l agar, and to which 1mol/l sodium hydroxide was added at the proportion of 1 ml/l) warmed at 50°C was added thereto, and the resulting mixture was stirred well. The mixture was then plated onto Ca-LB agar plates (which contains 10 g/l Bacto tryptone, 5 g/l Bacto yeast extract, 5 mmol/l calcium chloride, and 15 g/l agar, and to which 1 mol/l sodium hydroxide was added at the proportion of 1 ml/l; the diameter of the plates was 15 cm).

After the plates were incubated at 37°C for seven hours, soft agar portions were broken into fine pieces with a spreader and collected. The pieces were centrifuged at 1,500 x g and 4°C for ten minutes. 100 µl of chloroform was added to 1.5 ml of the supernatant, and the mixture was stored at 4°C overnight. On the next day, the liquid was centrifuged at 15,000 x g for two minutes, and the supernatant was stored as a phage stock at 4°C.

Cells of *E. coli* ΔnlpD-T strain were applied onto LB agar plates [which contains 10 g/l Bacto tryptone (Difco), 5 g/l Bacto yeast extract (Difco), 5 g/l sodium chloride, and 15 g/l agar, and to which 1 mol/l sodium hydroxide was added at the proportion of 1 ml/l]. The plates were incubated at 43°C overnight. Multiple colonies were simultaneously spotted onto both LB agar plates containing 100 µg/ml ampicillin and LB agar plates without ampicillin. Colonies grown only on the LB agar plates without ampicillin were selected to obtain a strain generated through removal of the temperature sensitive plasmid pKD46 from the *E. coli* ΔnlpD-T strain. The resulting strain was named *E. coli* BW25113ΔnlpD.

### [Example 2] Determination of ATP-producing activity of strain lacking the nlpD gene

ATP is a high-energy phosphate compound and a coenzyme that is essential for various reactions in organisms. Luciferase uses luciferin and ATP as substrates for luminescence. *E. coli* has ATP-producing activity. Thus, the ATP-producing activity of *E. coli* can be assessed using luminescence as an indicator.

### (1) Treatment of bacterial cells

Each of the *E. coli* BW25113ΔnlpD strain and its parental strain, *E. coli* BW25113, was cultured using 1 ml of liquid LB medium containing 3% glucose (LBG medium) with shaking at 30°C for 24 hours. Upon completion of culture, the turbidity was determined (measured at a wavelength of 590 nm; hereinafter referred to as OD590nm). When the turbidity value of BW25113 strain was taken as 100, the value of *E. coli* BW25113ΔnlpD strain was 109. 200 µl each of the resulting cultures was centrifuged to precipitate bacterial cells. The supernatant was discarded, and then the bacterial cells were washed once with 100 µl of 40 mmol/l Tris-HCl buffer (0°C, pH 7.4) and suspended in 30 µl of 40 mmol/l Tris-HCl buffer (0°C, pH 7.4). 30 µl of GT solution (40% glucose and 0.8% Triton X-100) was added to the resulting bacterial cell suspensions to prepare bacterial cell suspensions for the determination of the ATP-producing activity.

### (2) Determination of ATP-producing activity

10 µl of the bacterial cell suspension obtained as described above in (1) was added to 90 µl of a reaction solution (0.5 mmol/l D-luciferin, 1.25 µg/ml firefly luciferase, 5 mmol/l magnesium sulfate, 100 mmol/l ethylenediaminetetra sulfate, 1 mmol/l dithiothreitol, 0.4% Triton X-100, 15 mmol/l monopotassium phosphate, 25 mmol/l Tricine buffer (pH7.4)), and luminescence was measured at room temperature for three minutes. The luminescence value was determined using a plate reader of PerkinElmer. The luminescence value refers to a relative luminescence detection value that depends on the type of measurement device, and generally called as RLU.

Rates of ATP production were compared based on the time course changes of RLU: [RLU (3 min) - RLU (0 min)] / 3. When the rate of ATP production in *E. coli* BW25113 strain was taken as 100, the rate of ATP production in *E. coli* BW25113ΔnlpD strain was 182. This result shows that the ATP-producing activity was increased in the strain lacking the *nlpD* gene.

### [Example 3] Production of glutathione by strains lacking the nlpD gene

Glutathione is a peptidic substance consisting of three types of amino acids (glutamic acid, cysteine, and glycine). Glutathione is biosynthesized from the above three amino acids through condensation by the action of γ-glutamyl cysteine synthetase and glutathione synthetase. ATP is a coenzyme essential for this condensation. *E. coli* has ATP-producing activity, and thus the ATP-producing activity of *E. coli* can be assessed using the glutathione productivity as an indicator.

### (1) Obtaining the genes of the enzymes involved in glutathione biosynthesis

A mutant strain (*E*. *coli* RC912 strain) in which the inhibition of γ-glutamyl cysteine synthetase by glutathione is disabled is prepared by the method described in Japanese Published Unexamined Patent Application No. (JP-A) S58-020196 and J. Gen. Microbiol., 128, 1047-1052 (1982). The bacterial strain is also available as FERM BP-47.

Then, a *PstI* fragment containing the structural gene of γ-glutamyl cysteine synthetase in the chromosome of *E. coli* RC912 strain is cloned into plasmid pBR322 according to the method described in JP-A H02-031690 and Appl. Environ. Microbiol., 44, 1444-1448 (1982).

Then, a *Hin*dIII fragment containing the structural gene of glutathione synthetase in the chromosome of *E. coli* RC912 strain is cloned into plasmid pBR322 according to the method described in JP-A H02-031690 and Agric. Biol. Chem., 47, 1381-1383 (1983). Alternatively, the two types of cloned fragments may be inserted together into a single vector plasmid pBR325 according to the method described in JP-A H02-031690 and Bioprocess Technol., 19, 159-183 (1994) to prepare the same plasmid as pBR325-gshI·II or pGS500 described in JP-A H02-031690 or Bioprocess Technol., 19, 159-183 (1994). A strain carrying the plasmid is registered under the accession number FERM BP-337 in the NITE Patent Microorganisms Depositary, National Institute of Technology and Evaluation, Independent Administrative Institution. The plasmid was isolated from the strain FERM BP-337 and named pGS600. The plasmid was used in the experiments described below.

### (2) Introduction of plasmid into a strain lacking nlpD and its parental strain, and collection of bacterial cells

*E. coli* BW25113 strain and *E*. *coli* ΔnlpD-K strain, which lacks the *nlpD* gene and has *E. coli* BW25113 as its parental strain, were transformed with pGS600 by the calcium chloride method [Molecular Cloning, 3rd Ed., Cold Spring Harbor Laboratory Press, 2001]. Transformants were selected on LB agar plates containing 20 µg/ml of chloramphenicol, and they were named *E. coli* BW25113/pGS600 strain and *E. coli* BW25113ΔnlpD/pGS600 strain.

*E. coli* BW25113/pGS600 strain and E. *coli* BW25113ΔnlpD/pGS600 strain were cultured using 2.5 ml of liquid LB medium containing 3% glucose (LBG medium) with shaking at 30°C for 24 hours. 200 µl each of the resulting cultures was inoculated into Erlenmeyer flasks containing 20 ml of fresh LBG medium. The bacteria were cultured with shaking at 30°C for 24 hours. The turbidity (OD 590 nm) determined when the incubation was finished is shown in Table 1.

Each of the obtained cultures was centrifuged to precipitate bacterial cells. The cells were washed twice with 5 ml of 100 mmol/l Tris-HCl buffer (0°C, pH 7.4). To the washed bacterial cells, 200 µl of GT solution (20% glucose and 0.4% Triton X-100) was added, to prepare a bacterial cell suspension.

### (3) Glutathione production

100 µl of the bacterial cell suspension prepared as described above in (2) was added to 900 µl of a reaction solution (2% glucose, 20 mmol/l magnesium sulfate, 20 mmol/l dipotassium sulfate, 0.22 mmol/l oxidized nicotinamide adenine dinucleotide, 0.14 mmol/l flavin mononucleotide, 5 mmol/l glutamic acid, 5 mmol/l cysteine, 5 mmol/l glycine, 0.4% Triton X-100, 15 mmol/l monopotassium phosphate, and 200 mmol/l MOPS buffer (pH 7.8)). The resulting mixture was allowed to react with shaking at 30°C for 60 minutes. Glutathione accumulated in the reaction solution was quantified using a glutathione quantification kit (Dojindo Laboratories). The result is shown in Table 1.

**[Table 1]**

| Name of bacterial strain | Turbidity after culture (OD590nm) | Amount of accumulated glutathione (mmol/l) |
|---|---|---|
| BW25113/pGS600 | 2.1 | 0.66 |
| BW25113ΔnlpD/pGS600 | 2.9 | 1.37 |

As shown in Table 1, the glutathione productivity of the strain lacking the *nlpD* gene was higher than the parental strain by an extent exceeding the rate of increase in bacterial cell amount. This result shows that the enhancement of ATP-synthesizing activity of the strain lacking the *nlpD* gene can lead to an increase in substance productivity.

### [Example 4] Production of proline using strain lacking the nlpD gene

### (1) Preparation of strain lacking the proline-degrading enzyme gene putA

A DNA fragment containing a chloramphenicol resistance gene was amplified by PCR using pHSG398 plasmid DNA (purchased from TaKaRa Bio Inc.) as a template and a primer set of DNAs each consisting of the nucleotide sequence of SEQ ID NO: 8 or 9. 100 µl of a reaction solution containing 20 ng of purified plasmid DNA, 50 pmol each of the primer DNAs was prepared according to the instructions appended to EX-Taq (TaKaRa Bio Inc.). PCR was carried out using EX-Taq under the following conditions: keeping the temperature at 95°C for one minute, followed by 30 cycles of 94°C for 30 seconds, 64°C for 30 seconds, and 72°C for one minute, and subsequent incubation at 72°C for three minutes. The amplified DNA fragment was purified using QIA quick PCR Purification Kit (QIAGEN) to give 30 µl of a DNA solution.

Next, to digest a small amount of contaminating plasmid pHSG398, 10 U each of the restriction enzymes *Pst*I and *BgI*I was added to the DNA solution. After the mixture was kept at 37°C for two hours, the amplified DNA fragment was purified using QIA quick PCR Purification Kit and 30 µ of a DNA solution was obtained.

Then, a DNA fragment carrying the chloramphenicol resistance gene in the middle and nucleotide sequences homologous to the 5' and 3' ends of the *putA* gene at the both ends was amplified by PCR using 0.1 µl of the DNA solution as a template and a primer set of DNAs each consisting of the nucleotide sequence of SEQ ID NO: 10 or 11. 100 µl of a reaction solution containing 10 ng of template DNA and 50 pmol each of the primer DNAs was prepared according to the instructions appended to EX-Taq. PCR was carried out using EX-Taq under the following conditions: keeping the temperature at 95°C for one minute, followed by 30 cycles of 94°C for 30 seconds, 51°C for 30 seconds, and 72°C for one minute, and subsequent incubation at 72°C for three minutes. The amplified DNA fragment was purified in the same procedure as described above.

Then, *E*. *coli* BW25113/pKD46 strain was transformed with 1 µg of the DNA by electroporation in the same procedure as described in Example 1. The transformed cells were incubated in 1 ml of liquid SOC medium containing 2 mmol/l IPTG at 30°C for three hours, and then applied onto LB agar plates containing 50 µg/ml chloramphenicol (liquid LB medium containing 1.5% agar; hereinafter abbreviated as LBcm agar plates). The plates were incubated at 37°C overnight.

The grown strain was confirmed to be a strain in which the chloramphenicol resistance gene had been substituted for the *putA* gene in the chromosomal DNA of *E. coli* BW25113/pKD46 strain. The strain was named *E. coli* ΔputA-C.

Next, P1 phage was prepared from *E. coli* ΔputA-C strain by the same method as described in Example 1. *E. coli* W3110 strain was cultured in liquid Ca-LB medium at 30°C for four hours. A 200-µl aliquot of the culture was taken out, and 1 µl of the phage stock was added to it. After the mixture was kept at 37°C for ten minutes, 5 ml of liquid LB medium and 200 µl of 1 mol/l sodium citrate solution were added thereto. The mixture was stirred.

After ten minutes of centrifugation at 1500 x g and 25°C, the supernatant was discarded, and 1 ml of liquid LB medium and 10 µl of 1 mol/l sodium citrate solution were added to the precipitated cells. The cells were kept at 30°C for two hours. 100 µl of the solution was applied onto antibiotic medium 3 agar plates [0.15% meat extract, 0.15% yeast extract, 0.5% peptone, 0.1 % glucose, 0.35% sodium chloride, 0.132% dipotassium hydrogen phosphate (dipotassium phosphate), and 1.5% agar (pH 7.0)] containing 50 mg/l chloramphenicol. The plates were incubated at 30°C overnight.

Twenty four colonies formed were independently applied onto antibiotic medium 3 agar plates containing 50 mg/l chloramphenicol to confirm their drug sensitivity. *E. coli* W3110 strain to which the deletion of the *putA* gene [*ΔputA*::*Cm*] had been transduced was obtained by selecting chloramphenicol resistant strains. This strain was named *E*. *coli* W3110ΔputA.

### (2) Preparation of E. coli W3110ΔputA strain lacking the nlpD gene

The deletion of the *nlpD* gene [*ΔnlpD*::*tet*] derived from *E. coli* ΔnlpD-T strain was transduced into *E*. *coli* W3110ΔputA strain using a stock of P1 phage derived from *E*. *coli* ΔnlpD-T strain prepared as described in Example 1 by the same method as the phage-mediated transduction described above in (1). A strain showing resistance to 20 mg/l tetracycline was selected as a transduced strain and named *E*. *coli* W3110ΔputAΔnlpD.

### (3) Preparation of plasmid carrying desensitized proBA operon

### (i) Preparation of plasmid carrying mutant proBA operon

A 4-kbp DNA fragment carrying the *proBA* operon was amplified by PCR using chromosomal DNA of *E. coli* W3110 strain as a template and a primer set of DNAs each consisting of the nucleotide sequence of SEQ ID NO: 12 or 13. 25 µl of a reaction solution containing 10 ng of chromosomal DNA and 20 pmol each of primer DNAs was prepared according the instructions appended to LA-Taq. PCR was carried out using LA-Taq under the following conditions: maintaining the temperature at 94°C for three minutes, followed by 30 cycles of 94°C for 15 seconds, 55°C for 20 seconds, and 68°C for four minutes, and subsequent incubation at 72°C for ten minutes.

After amplification of a DNA fragment of interest was confirmed by agarose gel electrophoresis, the DNA fragment was excised from the gel and purified. The plasmid pPCR proBA was prepared by cloning the fragment into the plasmid pPCR-Script Amp using PCR-Script Cloning Kit (QIAGEN). The point mutation proB74 [Gene, 64, 199-205 (1988)] was introduced into *proB* of pPCR proBA using QuickChange Kit (QIAGEN) according to the instructions appended to the kit. In this manner, the plasmid pPCR proB74A was prepared. The nucleotide sequence of the primer DNA used to introduce the point mutation is shown in SEQ ID NO: 14.

A proB74A gene with an addition of an SD sequence and restriction sites for *Bam*HI and *Hin*dIII was amplified by PCR using the plasmid pPCR proB74A as a template and DNAs each consisting of the nucleotide sequence of SEQ ID NO: 15 or 16 as primers. 25 µl of a reaction solution containing 100 ng of plasmid DNA and 20 pmol each of primer DNAs was prepared, and PCR was carried out using Pyrobest (TaKaRa Bio Inc.) under the following conditions: keeping the temperature at 98°C for two minutes, followed by 25 cycles of 98°C for ten seconds, 55°C for 30 seconds, and 72°C for 2.5 minutes.

The amplified fragment was purified and cleaved by the restriction enzymes *Bam*HI and *Hin*dIII. The vector portion of pQE80 (QIAGEN) treated with the same restriction enzymes was fractionated by agarose electrophoresis, and then excised and purified. The two fragments were ligated together using TaKaRa Ligation Kit to prepare a recombinant DNA. *E. coli* DH10B (Invitrogen) was transformed with the recombinant DNA, and applied onto LB agar plates containing 50 µg/ml ampicillin. A plasmid was purified from colonies formed. The obtained plasmid carries the desensitized proline biosynthetic gene that is inducible by IPTG This plasmid was named pQE80proB74A.

*E. coli* W3110ΔputA and *E. coli* W3110ΔputAΔnlpD strains were independently transformed with pQE80proB74A by the calcium chloride method [Molecular Cloning, 3rd Ed., Cold Spring Harbor Laboratory Press, 2001]. Transformants were selected using LB agar plates containing 50 µg/ml ampicillin. Each transformant was named *E. coli* ΔputA/pQE80proB74A strain and *E. coli* ΔputAΔnlpD/pQE80proB74A strain.

The above transformed strains were independently inoculated in a test tube containing 5 ml of liquid Med4 medium (2% glucose, 1% polypeptone, 0.5% Bacto yeast extract, 1% sodium chloride, and 2% calcium carbonate), and cultured with shaking at 30°C for 20 hours. Thus, cultures were obtained.

A 300-µl aliquot of each culture was inoculated in a 300-ml Erlenmeyer flask containing 30 ml of liquid Med7 medium [4% glucose, 0.8% peptone, 0.1% potassium dihydrogen phosphate (monopotassium phosphate), 0.05% magnesium sulfate heptahydrate, 0.002% calcium chloride, 2% calcium carbonate, 1% ammonium sulfate, 0.2% sodium chloride, and 0.03% ferrous sulfate]. The transformants were cultured with shaking at 30°C. When the turbidity (OD660nm) reached 0.7, IPTG was added at a final concentration of 50 µmol/l.

The culture was finished 41 hours after the start of incubation. An aliquot was sampled from the culture and centrifuged. The supernatant was 1000 times diluted and measured for the amount of proline accumulated in the culture using the direct amino acid analysis system DXa-500 of DIONEX. The result is shown in Table 2.

**[Table 2]**

| Name of bacterial strain | Turbidity after culture (OD660nm) | Amount of accumulated proline (g/l) |
|---|---|---|
| ΔputA/pQE80proB74A | 9.2 | 6.8 |
| ΔputAΔnlpD/pQE80proB74A | 9.0 | 12.0 |

As shown in Table 2, the proline productivity of the strain lacking the *nlpD* gene was 1.7 or more times higher than that of the control strain. This result suggests that the strain lacking the *nlpD* gene can be used not only in the production of substances that directly involves ATP in the biosynthetic pathways, but also in the production of various substances using microorganisms and cells, wherein the production indirectly involves ATP.

### Industrial Applicability

According to the present invention, useful substances can be efficiently produced.

### Sequence Listing Free Text

SEQ ID NO: 3 - Note for the artificial sequence; synthetic DNA
SEQ ID NO: 4 - Note for the artificial sequence; synthetic DNA
SEQ ID NO: 5 - Note for the artificial sequence; synthetic DNA
SEQ ID NO: 6 - Note for the artificial sequence; synthetic DNA
SEQ ID NO: 7 - Note for the artificial sequence; synthetic DNA
SEQ ID NO: 8 - Note for the artificial sequence; synthetic DNA
SEQ ID NO: 9 - Note for the artificial sequence; synthetic DNA
SEQ ID NO: 10 - Note for the artificial sequence; synthetic DNA
SEQ ID NO: 11 - Note for the artificial sequence; synthetic DNA
SEQ ID NO: 12 - Note for the artificial sequence; synthetic DNA
SEQ ID NO: 13 - Note for the artificial sequence; synthetic DNA
SEQ ID NO: 14 - Note for the artificial sequence; synthetic DNA
SEQ ID NO: 15 - Note for the artificial sequence; synthetic DNA
SEQ ID NO: 16 - Note for the artificial sequence; synthetic DNA

## Claims

1. A process for producing a useful substance, which comprises culturing a microorganism that lacks from its chromosomal DNA all or part of a gene encoding a protein comprising the amino acid sequence of SEQ ID NO: 1 or a gene encoding a protein that has 80% or more homology to the amino acid sequence of SEQ ID NO: 1 in a medium so as to produce and accumulate the useful substance in the culture, and recovering the useful substance from the culture.

2. A process for producing a useful substance, which comprises allowing a culture or a treated culture of a microorganism that lacks from its chromosomal DNA all or part of a gene encoding a protein comprising the amino acid sequence of SEQ ID NO: 1 or a gene encoding a protein that has 80% or more homology to the amino acid sequence of SEQ ID NO: 1, an enzyme having an activity to produce a useful substance using ATP as an energy source or a culture or a treated culture of a cell having the activity, a carbon source, and a precursor substance of the useful substance to coexist in a medium so as to produce and accumulate the useful substance in the medium, and recovering the useful substance from the medium.

3. The process according to claim 1 or 2, wherein the gene encoding a protein comprising the amino acid sequence of SEQ ID NO: 1 has the nucleotide sequence of SEQ ID NO: 2.

4. The process according to any one of claims 1 to 3, wherein the ability of the microorganism or cell to produce the useful substance has been improved by a method selected from the following (1) to (5):
(1) a method of partially or completely releasing at least one of the mechanisms that regulate the biosynthesis of the useful substance;
(2) a method of enhancing expression of at least one of the enzymes involved in the biosynthesis of the useful substance;
(3) a method of increasing the copy number of at least one of the enzyme genes involved in the biosynthesis of the useful substance;
(4) a method of partially or completely blocking at least one of the branched metabolic pathways of the biosynthetic pathway of the useful substance to metabolites other than the useful substance; and
(5) a method of selecting a cell strain that is more resistant to an analog of the useful substance as compared with a wild type strain.

5. The process according to any one of claims 1 to 4, wherein the microorganism belongs to the genus *Azotobacter, Erwinia, Escherichia, Klebsiella, Methylobacillus, Pseudomonas,* or *Salmonella.*

6. The process according to any one of claims 1 to 5, wherein the microorganism is a microorganism of the species *Azotobacter vinelandii, Erwinia carotovora, Escherichia coli, Klebsiella pneumoniae, Methylobacillusflagellatus, Pseudomonas fluorescens, Pseudomonas putida,* or *Salmonella thypimurium.*

7. The process according to any one of claims 1 to 6, wherein the useful substance is selected from the group consisting of a protein, peptide, amino acid, nucleic acid, vitamin, sugar, sugar alcohol, alcohol, organic acid, biologically active low-molecular-weight compound, and lipid.


<110> KYOWA HAKKO KOGYO CO., LTD.

<120> Process for producing useful substance

<130> K 10009 EP

<150> 2005-09-29
<151> JP2005-284540

<160> 16

<170> PatentIn Ver. 2.1

<210> 1
<211> 379
<212> PRT
<213> Escherichia coli

<400> 1
Met Ser Ala Gly Ser Pro Lys Phe Thr Val Arg Arg Ile Ala Ala Leu
  1               5                  10                  15

Ser Leu Val Ser Leu Trp Leu Ala Gly Cys Ser Asp Thr Ser Asn Pro
             20                  25                  30

Pro Ala Pro Val Ser Ser Val Asn Gly Asn Ala Pro Ala Asn Thr Asn
         35                  40                  45

Ser Gly Met Leu Ile Thr Pro Pro Pro Lys Met Gly Thr Thr Ser Thr
     50                  55                  60

Ala Gln Gln Pro Gln Ile Gln Pro Val Gln Gln Pro Gln Ile Gln Ala
 65                  70                  75                  80

Thr Gln Gln Pro Gln Ile Gln Pro Val Gln Pro Val Ala Gln Gln Pro
                 85                  90                  95

Val Gln Met Glu Asn Gly Arg Ile Val Tyr Asn Arg Gln Tyr Gly Asn
            100                 105                 110

Ile Pro Lys Gly Ser Tyr Ser Gly Ser Thr Tyr Thr Val Lys Lys Gly
        115                 120                 125

Asp Thr Leu Phe Tyr Ile Ala Trp Ile Thr Gly Asn Asp Phe Arg Asp
    130                 135                 140

Leu Ala Gln Arg Asn Asn Ile Gln Ala Pro Tyr Ala Leu Asn Val Gly
145                 150                 155                 160

Gln Thr Leu Gln Val Gly Asn Ala Ser Gly Thr Pro Ile Thr Gly Gly
                165                 170                 175

Asn Ala Ile Thr Gln Ala Asp Ala Ala Glu Gln Gly Val Val Ile Lys
            180                 185                 190

Pro Ala Gln Asn Ser Thr Val Ala Val Ala Ser Gln Pro Thr Ile Thr
        195                 200                 205

Tyr Ser Glu Ser Ser Gly Glu Gln Ser Ala Asn Lys Met Leu Pro Asn
    210                 215                 220

Asn Lys Pro Thr Ala Thr Thr Val Thr Ala Pro Val Thr Val Pro Thr
225                 230                 235                 240

Ala Ser Thr Thr Glu Pro Thr Val Ser Ser Thr Ser Thr Ser Thr Pro
                245                 250                 255

Ile Ser Thr Trp Arg Trp Pro Thr Glu Gly Lys Val Ile Glu Thr Phe
            260                 265                 270

Gly Ala Ser Glu Gly Gly Asn Lys Gly Ile Asp Ile Ala Gly Ser Lys
        275                 280                 285

Gly Gln Ala Ile Ile Ala Thr Ala Asp Gly Arg Val Val Tyr Ala Gly
    290                 295                 300

Asn Ala Leu Arg Gly Tyr Gly Asn Leu Ile Ile Ile Lys His Asn Asp
305                 310                 315                 320

Asp Tyr Leu Ser Ala Tyr Ala His Asn Asp Thr Met Leu Val Arg Glu
                325                 330                 335

Gln Gln Glu Val Lys Ala Gly Gln Lys Ile Ala Thr Met Gly Ser Thr
            340                 345                 350

Gly Thr Ser Ser Thr Arg Leu His Phe Glu Ile Arg Tyr Lys Gly Lys
        355                 360                 365

Ser Val Asn Pro Leu Arg Tyr Leu Pro Gln Arg
    370                 375

<210> 2
<211> 1440
<212> DNA
<213> Escherichia coli

<220>
<221> CDS
<222> (301)..(1437)
<223>

<400> 2
ctgcgctaat gacgcagctg gacgaaggcg ggattctcgt cttacccgta ggggaggagc 60

accagtattt gaaacgggtg cgtcgtcggg gaggcgaatt tattatcgat accgtggagg 120

ccgtgcgctt tgtcccttta gtgaagggtg agctggctta aaacgtgagg aaatacctgg 180

atttttcctg gttattttgc cgcaggtcag cgtatcgtga acatcttttc cagtgttcag 240

tagggtgcct tgcacggtaa ttatgtcact ggttattaac caatttttcc tgggggataa 300

atg agc gcg gga agc cca aaa ttc acc gtt cgc cgc att gcg gct ttg   348

Met Ser Ala Gly Ser Pro Lys Phe Thr Val Arg Arg Ile Ala Ala Leu
1               5                   10                  15

tca ctg gtt tcg cta tgg ctg gca ggc tgt tct gac act tca aat cca     396
Ser Leu Val Ser Leu Trp Leu Ala Gly Cys Ser Asp Thr Ser Asn Pro
            20                  25                  30

ccg gca ccg gtc agc tcc gtt aat ggc aat gcg cct gca aat act aat     444
Pro Ala Pro Val Ser Ser Val Asn Gly Asn Ala Pro Ala Asn Thr Asn
        35                  40                  45

tct ggt atg ttg att acg ccg ccg ccg aaa atg ggg acg acg tct aca     492
Ser Gly Met Leu Ile Thr Pro Pro Pro Lys Met Gly Thr Thr Ser Thr
    50                  55                  60

gcg cag caa ccg caa att cag ccg gtg cag cag cca caa att cag gct     540
Ala Gln Gln Pro Gln Ile Gln Pro Val Gln Gln Pro Gln Ile Gln Ala
65                  70                  75                  80

act caa caa ccg caa atc cag cca gtg cag cca gta gct cag cag ccg     588
Thr Gln Gln Pro Gln Ile Gln Pro Val Gln Pro Val Ala Gln Gln Pro
                85                  90                  95

gta cag atg gaa aac gga cgc atc gtc tat aac cgt cag tat ggg aac     636
Val Gln Met Glu Asn Gly Arg Ile Val Tyr Asn Arg Gln Tyr Gly Asn
            100                 105                 110

att ccg aaa ggc agt tat agc ggc agt acc tat acc gtg aaa aaa ggc     684
Ile Pro Lys Gly Ser Tyr Ser Gly Ser Thr Tyr Thr Val Lys Lys Gly
        115                 120                 125

gac aca ctt ttc tat atc gcc tgg att act ggc aac gat ttc cgt gac     732
Asp Thr Leu Phe Tyr Ile Ala Trp Ile Thr Gly Asn Asp Phe Arg Asp
    130                 135                 140

ctt gct cag cgc aac aat att cag gca cca tac gcg ctg aac gtt ggt     780
Leu Ala Gln Arg Asn Asn Ile Gln Ala Pro Tyr Ala Leu Asn Val Gly
145                 150                 155                 160

cag acc ttg cag gtg ggt aat gct tcc ggt acg cca atc act ggc gga     828
Gln Thr Leu Gln Val Gly Asn Ala Ser Gly Thr Pro Ile Thr Gly Gly
                165                 170                 175

aat gcc att acc cag gcc gac gca gca gag caa gga gtt gtg atc aag     876
Asn Ala Ile Thr Gln Ala Asp Ala Ala Glu Gln Gly Val Val Ile Lys
            180                 185                 190

cct gca caa aat tcc acc gtt gct gtt gcg tcg caa ccg aca att acg     924
Pro Ala Gln Asn Ser Thr Val Ala Val Ala Ser Gln Pro Thr Ile Thr
        195                 200                 205

tat tct gag tct tcg ggt gaa cag agt gct aac aaa atg ttg ccg aac     972
Tyr Ser Glu Ser Ser Gly Glu Gln Ser Ala Asn Lys Met Leu Pro Asn
    210                 215                 220

aac aag cca act gcg acc acg gtc aca gcg cct gta acg gta cca aca     1020
Asn Lys Pro Thr Ala Thr Thr Val Thr Ala Pro Val Thr Val Pro Thr

225                 230                 235                 240

gca agc aca acc gag ccg act gtc agc agt aca tca acc agt acg cct     1068
Ala Ser Thr Thr Glu Pro Thr Val Ser Ser Thr Ser Thr Ser Thr Pro
                245                 250                 255

atc tcc acc tgg cgc tgg ccg act gag ggc aaa gtg atc gaa acc ttt     1116
Ile Ser Thr Trp Arg Trp Pro Thr Glu Gly Lys Val Ile Glu Thr Phe
            260                 265                 270

ggc gct tct gag ggg ggc aac aag ggg att gat atc gca ggc agc aaa     1164
Gly Ala Ser Glu Gly Gly Asn Lys Gly Ile Asp Ile Ala Gly Ser Lys
        275                 280                 285

gga cag gca att atc gcg acc gca gat ggc cgc gtt gtt tat gct ggt     1212
Gly Gln Ala Ile Ile Ala Thr Ala Asp Gly Arg Val Val Tyr Ala Gly
    290                 295                 300

aac gcg ctg cgc ggc tac ggt aat ctg att atc atc aaa cat aat gat     1260
Asn Ala Leu Arg Gly Tyr Gly Asn Leu Ile Ile Ile Lys His Asn Asp
305                 310                 315                 320

gat tac ctg agt gcc tac gcc cat aac gac aca atg ctg gtc cgg gaa     1308
Asp Tyr Leu Ser Ala Tyr Ala His Asn Asp Thr Met Leu Val Arg Glu
                325                 330                 335

caa caa gaa gtt aag gcg ggg caa aaa ata gcg acc atg ggt agc acc     1356
Gln Gln Glu Val Lys Ala Gly Gln Lys Ile Ala Thr Met Gly Ser Thr
            340                 345                 350

gga acc agt tca aca cgc ttg cat ttt gaa att cgt tac aag ggg aaa     1404
Gly Thr Ser Ser Thr Arg Leu His Phe Glu Ile Arg Tyr Lys Gly Lys
        355                 360                 365

tcc gta aac ccg ctg cgt tat ttg ccg cag cga taa                     1440
Ser Val Asn Pro Leu Arg Tyr Leu Pro Gln Arg
    370                 375


<210> 3
<211> 34
<212> DNA
<213> Artificial Sequence

<400> 3
GAAGTTCCTA TACTTTCTAG AGAATAGGAA CTTC                               34


<210> 4
<211> 48
<212> DNA
<213> Artificial Sequence

<400> 4
attcccctgc tcgcgcaggc tgggtaggcc aatttattgc tatttacc                48

<210> 5
<211> 47
<212> DNA
<213> Artificial Sequence

<400> 5
ttatttgccg actaccttgg tgatcagctc taatgcgctg ttaatca 47

<210> 6
<211> 95
<212> DNA
<213> Artificial Sequence

<400> 6
acggtaatta tgtcactggt tattaaccaa tttttcctgg gggataaatg attccgggga 60

tccgtcgacc ttatttgccg actaccttgg tgatc 95

<210> 7
<211> 95
<212> DNA
<213> Artificial Sequence

<400> 7
cattcaagca aaagcctggt tccgccgatt tatcgctgcg gcaaataacg tgtaggctgg 60

agctgcttcg attcccctgc tcgcgcaggc tgggt 95

<210> 8
<211> 24
<212> DNA
<213> Artificial Sequence

<400> 8
acggaagatc acttcgcaga ataa 24

<210> 9
<211> 24
<212> DNA
<213> Artificial Sequence

<400> 9
attaattgcg ttgcgctcac tgcc 24

<210> 10
<211> 74
<212> DNA
<213> Artificial Sequence

<400> 10
atgggaacca ccaccatggg ggttaagctg gacgacgcga atgagacgtt gatcggcacg 60
taagaggttc caac 74

<210> 11
<211> 56
<212> DNA
<213> Artificial Sequence

<400> 11
ttaacctata gtcattaagc tggcgttacc gccagcggca tttctgccat tcatcc     56


<210> 12
<211> 25
<212> DNA
<213> Artificial Sequence

<400> 12
tcgtatttca gacctgttgc ccatg                                       25


<210> 13
<211> 23
<212> DNA
<213> Artificial Sequence

<400> 13
tgcgaagccg acacccttgg cag                                         23


<210> 14
<211> 40
<212> DNA
<213> Artificial Sequence

<400> 14
aaatgctgct gacccgtgct aatatggaag accgtgaacg                       40


<210> 15
<211> 55
<212> DNA
<213> Artificial Sequence

<400> 15
cgcggaatca ttaaagagga gaaattaacc atgagtgaca gccagacgct ggtgg      55


<210> 16
<211> 34
<212> DNA
<213> Artificial Sequence

<400> 16
cccaagcttt tacgcacgaa tggtgtaatc acca                             34